# EUROPEAN PATENT SPECIFICATION

(11) **EP 1 898 855 B1**
(45) Date of publication and mention of the grant of the patent: **11.06.2014**
(21) Application number: 05754853.9
(22) Date of filing: 28.06.2005
(51) Int. Cl.: A61F 13/20, A61F 13/15

(54) **TAMPON**
TAMPON
TAMPON

(43) Date of publication of application: 19.03.2008
(73) Proprietor: SCA Hygiene Products AB, 405 03 Göteborg (SE)
(72) Inventor: MATHEWS, Steve, Sandringham, VIC 3191 (AU)
(74) Representative: Andersson, Per Rune
(86) International application number: PCT/SE2005/001024
(87) International publication number: WO 2007/001216

(56) References cited:
- EP-A1- 0 301 874
- EP-A1- 1 108 408
- US-A1- 2003 135 180
- US-A1- 2003 229 328

## Description

### TECHNICAL FIELD

The invention pertains to a tampon having an insertion end and a withdrawal end, and a withdrawal string extending from the withdrawal end, the tampon comprising a fibrous absorbent tampon body, the tampon body comprising an inner core and an outer absorbent structure surrounding the inner core, the inner core having a higher density than the outer absorbent structure.

### BACKGROUND ART

Menstrual tampons for intra-vaginal use have been known and used for a very long time. Since tampons are worn internally, they are considered to be discrete and mostly very comfortable to wear, lacking the occluding plastic backing of external protection devices such as sanitary napkins.

The tampons are commonly made from fibres, usually cellulosic fibres such cotton fibres or regenerated cellulose fibres, such as viscose.

For several reasons, it is of interest to increase the absorption capacity of the fibrous material used for the manufacturing of tampons. Fibres having greater absorption capacity allow the tampons to be made smaller. The benefits of making the tampons smaller include reduced material costs, consumer preference to use smaller tampons, and savings and consumer preference for more compact packaging of the tampons.

In order to meet the demand for fibres having higher absorbency, multilimbed regenerated cellulosic fibres have been developed. Multilimbed viscose fibres have a cross-section with three or more lobes or limbs and are known to have higher absorbency than corresponding non-limbed viscose fibres. One example of viscose fibres having a triangular cross-section and improved absorbency is disclosed In WO 2004/085720.

A further example is disclosed by US2003/229328 which discloses a tampon having an insertion end and withdrawal string extending from the withdrawal end. The tampon comprises a fibrous absorbent tampon body comprising multi-limbed viscose fibres. The tampon body comprises an inner core and an outer absorbent structure.

However, the multilimbed regenerated cellulose fibres have proven to be less suitable for direct use in conventional tampon production since they cannot readily be compressed into a state that ensures rigidity of the tampon. In order to be easy to manipulate and to insert into the vaginal cavity without collapsing, a tampon needs to have a degree of rigidity. Excessive compression of multilobal fibres has been found to proportionally reduce the effective absorbency of the fibres. Hence, even if the compression problem were to be solved, the absorbency of the material would be compromised.

The simplest and conventional approach to resolving the stability issue is to blend the high-absorbency multi-lobal fibres with non-limbed viscose fibres to gain back the stability of the product. Again, this compromises the absorbency issue. US 2002/0151859, US 2003/0229328, WO 01/43679 and WO 2004/000184 disclose tampon cores and the manufacture of tampon cores comprising blends of multi-lobal fibres and non-lobal fibres.

Hence, it is an object of the present invention to provide a vaginal tampon having improved absorbency but still exhibits sufficient rigidity to allow easy handling and insertion of the tampon.

It is a further object of the invention to provide a vaginal tampon having a soft surface and good expansion properties.

### DISCLOSURE OF INVENTION

The present invention offers a tampon comprising multi-limbed viscose fibres that are better utilized than in previously known tampons containing such fibres. The tampon has an inner core that is made of a fibrous blend comprising 0 - 25% by weight of multilimbed viscose fibres and an outer absorbent structure surrounding the inner core and being made of a fibrous blend comprising 30-100% by weight of multilimbed viscose fibres.

The inner core is more compressible than the outer absorbent structure and retains compression better than the outer absorbent structure. The inner core is preferably predominantly made of highly compressible and compression-retaining material such as non-limbed viscose fibres. However, alternative readily compressible materials such as cotton fibres, cellulose pulp, peat moss, etc. may be used alone or in combination. It is also conceivable to use bonded fibrous materials containing bonding agents such as thermoplastic particles or thermoplastic bonding fibres. The inner core can include non-absorbent material such as synthetic fibres and can be completely non-absorbent or can have only a small degree of absorbency. However, for most purposes it is preferred that the inner core is absorbent.

The outer absorbent structure contains a relatively large proportion of multi-limbed viscose fibres. The outer absorbent structure is less dense than the inner core. This implies that the outer absorbent structure is softer and has a more open pore structure than the inner core. Preferably, the outer absorbent structure is free from binders such as adhesive or thermoplastic fibres or particles and is only bonded by mechanical entangling of the constituting fibres and by hydrogen bonds.

Suitable multilimbed viscose fibres are the trilobal fibres provided by Kelheim, under the trade name Galaxy. The fibres are preferably staple length fibres having at least three limbs and can have a Y-, X-, H-, or T-, cross-sectional shape with a symmetrical Y-shape being preferred.

Due to the inner core being more compressed or at least denser than the outer absorbent structure, the tampon has the requisite rigidity and is easy to grip and insert into the vaginal cavity without bunching or bending. Moreover, the less dense outer absorbent structure provides the principal absorption capacity. The lower density in the outer absorbent structure and the superior expansion properties of the multi-limbed viscose fibres ensures rapid initial absorption of menses and makes the outer part of the tampon soft and comfortable both during insertion and use.

The inner core comprises a blend of 0 - 25% by weight of multilimbed viscose fibres and 75 - 100 % of non-limbed viscose fibres. The tampon body can be made from 100 % by weight of viscose fibres or can comprise additional components such as binders, superabsorbents and non-absorbent fibres. Any chemical agents added to the surface of the fibres to improve wettability and/or processability of the fibres are included in the fibre weight.

The multilimbed viscose fibres preferably have an absorbent capacity of at least 5 g/g and preferably at least 5.5 g/g.

The inner core preferably has a density of 0.40 - 0.60 g/cm³, more preferably 0.50 - 0.55 g/cm³. The outer absorbent structure preferably has a density of 0.30 - 0.45 g/cm³, more preferably 0.35 - 0.40 g/cm³.

In accordance with a preferred embodiment of the invention, both the inner core and the outer core are absorbent, the inner core having an absorption capacity of at least 3,5 g/g and preferably at least 4 g/g and the outer absorbent structure has an absorption capacity of at least 5 g/g and preferably at least 5.5 g/g.

The inner core is predominantly made from non-limbed cellulosic fibres such as non-limbed viscose fibres, cotton fibres or cellulose fluff pulp fibres and the outer core is preferably predominantly made from multi-limbed regenerated cellulose fibres. Hence, the inner core may comprise at least 80% non-limbed cellulosic fibres and the outer absorbent structure may comprise at least 80% multi-limbed viscose fibres.

It may be beneficial if the outer absorbent structure is radially compressed so that it exhibits at least three longitudinally extending grooves and at least three longitudinally extending ridges separating the grooves.

The tampon according to the invention may be provided with a liquid permeable cover sheet that is arranged on the surface of the absorbent body. The cover is preferably of a material exhibiting low friction against the mucous membranes in the vaginal cavity. The tampon preferably has a generally cylindrical shape and can have a rounded insertion end. The cover sheet may be arranged to cover only the length of the tampon, leaving one or both ends free from cover material. Alternatively, the absorbent body can be completely covered by the liquid permeable cover sheet or be covered only at the insertion end or at the withdrawal end or both. The cover sheet facilitates insertion and removal of the tampon, and prevents linting. Suitable cover materials include nonwovens, nettings and perforated plastic films. The cover material may be hydrophilic or hydrophobic. A hydrophilic cover material may be a non-absorbent polymer material with a hydrophilic surface finish. It is also possible to use cover materials that are inherently hydrophilic and more or less absorbent. Usually, it is desirable to use cover materials having at least some degree of absorbency in order for liquid to be retained in the cover sheet so that drying out of the mucous membranes in the vaginal cavity is avoided.

The surface of the tampon may also be treated with friction reducing agents such as oils, waxes or similar.

In order to improve leakage security, a liquid barrier can be arranged at the withdrawal end of the tampon. The barrier can be a coating, for instance a coating of a thermoplastic material or a wax or can be provided by fusing of a thermoplastic component in a cover material extending over the withdrawal end.

The two parts of the tampon body may be formed and compressed simultaneously. After having been exposed to a particular applied compression force, the inner core will then retain the compressed state to a greater degree than the outer absorbent structure. The resiliency and low fibre-to-fibre-friction of the multi-limbed fibres which make up a greater part of the outer absorbent structure will cause the outer absorbent structure to spring back to a less compressed state.

The tampon body can be made by forming a tampon blank of a first fibrous band of carded fibres overlaid by a second band of carded fibres. Optionally, the tampon blank includes a piece of cover material attached to the second band of carded fibres. The first and second bands and the cover material are preferably attached to each other by means of a thermoplastic adhesive binder, a latex binder, by welding or merely by compression and hydrogen bonding. The fibre composite is then rolled up with the first fibrous band on the inside of the roll and the second fibrous band on the outside so that the first fibrous band forms an inner core and the second fibrous band forms an outer absorbent structure. If a cover is attached to the blank, it will be wound up on the outside of the outer absorbent structure. A withdrawal string may be fixated internally in the roll by being wound between one or more layers in the roll. After rolling up the blank, the resulting pledget is radially compressed to form the finished tampon body.

In order to increase the frictional forces between the inner absorbent core and the outer absorbent structure, the inner absorbent core can have a higher concentration of multi-limbed viscose fibres in an outer portion of the inner absorbent core than in an interior portion of the inner absorbent core. By increasing the frictional forces between the component parts of the tampon body, the risk of an internally wound withdrawal string slipping out between the layers of the tampon body is reduced.

It is not necessary that a cover be applied to the tampon at the same time as the tampon body is formed. Hence, it is conceivable to form a cylindrical tampon body and to apply the cover before or after compression of the tampon body.

Similarly, the withdrawal string may be attached to the tampon body after it has been formed.

The tampon may be provided with a leakage protecting barrier at the withdrawal end. One way of accomplishing such a barrier is by axially compressing the withdrawal end of the tampon and simultaneously fusing thermoplastic material in a cover extending over the withdrawal end as disclosed in WO 94/15565.

Alternatively, the tampon body may be made by first forming and compressing the inner core, than applying the outer absorbent structure to the outside of the inner core and compressing the resulting tampon pledget.

Compression of the tampon body may be made, for instance, as disclosed in US 5,592,725, US 2002/0151859, US 6,310,269, WO 97/23185, EP 611 562, WO 2004/000184 or WO 01/43679. These compression methods result in a tampon body having longitudinally extending ribs and grooves on the outer surface.

### BRIEF DESCRIPTION OF DRAWINGS

The invention will in the following be described in greater detail, with reference to the figures that are shown on the appended drawings. In the drawings:
- Fig. 1: shows a tampon in accordance with a first embodiment of the invention.
- Fig. 2: shows a cross-section through the tampon in Fig. 1 taken along the line II - II.
- Fig. 3: shows a trilobal fibre.
- Fig. 4: shows a tampon blank useful for the production of a tampon in accordance with the invention.
- Fig. 5: shows the tampon blank in Fig..4 after it has been wound into an uncompressed roll.
- Fig. 6: a cross-section through the tampon roll in Fig. 5 taken along the line VI - VI.

### EMBODIMENTS OF THE INVENTION

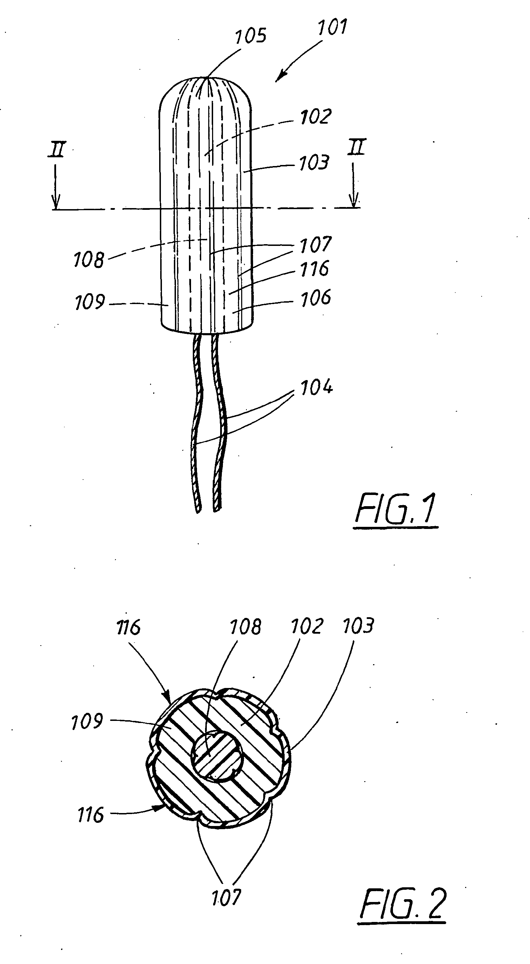

The tampon 101 shown in Figs. 1 and 2 comprises an absorption body 102 completely enclosed in a liquid permeable cover 103 and provided with two withdrawal strings 104. The tampon 101 has an elongate generally cylindrical shape with a rounded insertion end 105 and a withdrawal end 106. The withdrawal strings 104 protrude from the withdrawal end 106.

The absorption body 102 is a layered absorbent fibrous structure and has been compressed into a roughly cylindrical shape. As is common in the art and as shown in Fig. 1, the tampon is provided with longitudinally extending ridges 116 separated by compressed grooves 107 that aid liquid distribution along the length of the tampon.

In accordance with the invention, the absorption body 102 comprises an inner core 108 and an outer absorbent structure 109 radially surrounding the inner core. The inner core is denser than the outer core and provides the tampon with sufficient rigidity to allow the tampon to be easily inserted into the vaginal cavity without bunching or bending. The core 108 is predominantly made of fibres that are compressible and that, after compression, retain their compressed state, at least as long as the tampon is dry.

Suitable materials for the inner core 108 are cellulose fibres such as non-limbed viscose, cotton and cellulose fluff pulp. The inner core 108 may comprise a limited amount of multilimbed viscose fibres, up to a maximum of 25% by weight of multi-limbed viscose fibres. The multilimbed viscose fibres may be substantially homogeneously blended with the other components of the inner core 108 or may be concentrated to certain parts of the core 108, such as the outer periphery of the core 108. The inner core 108 may also comprise a binder such as thermoplastic fibres or particles. Although it is preferred that the inner core 108 be absorbent, it is conceivable to use non-absorbent materials or materials with only a limited degree of absorbency for the inner core. Hence, in applications where the main function of the inner core 108 is to provide rigidity to the tampon, the inner core may be a mass of thermoplastic, fused fibres such as polyethylene, polypropylene, polyester or bicomponent fibres.

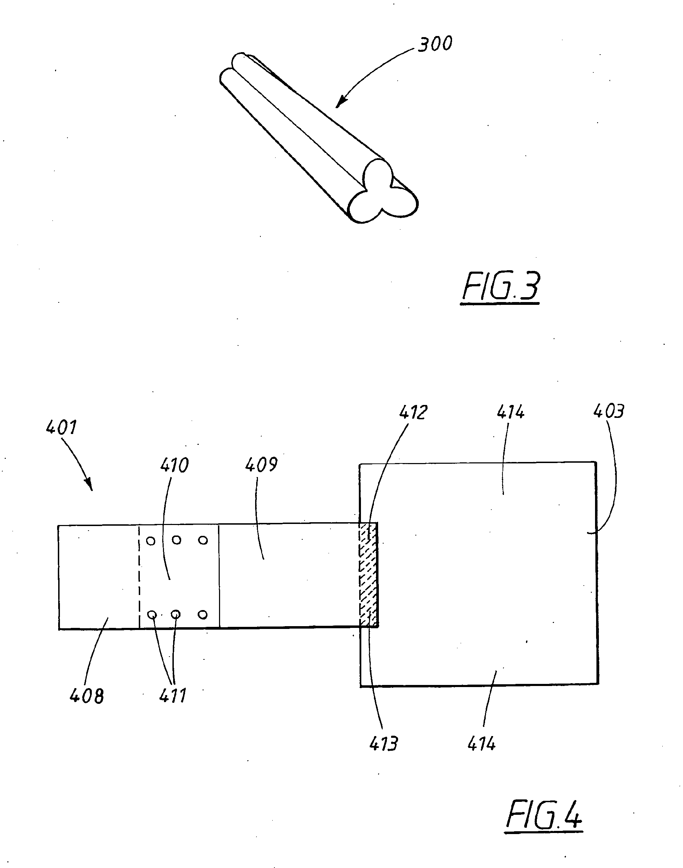

The outer absorbent structure 109 is made of a fibrous blend comprising 30 - 100% by weight of multilimbed viscose fibres. Multilimbed viscose fibres are viscose fibres having a cross-section with at least three limbs or lobes. One example of useful multilimbed fibres are the trilobal fibres provided by Kelheim, under the trade name Galaxy. The multilimbed fibres are preferably staple length fibres having at least three limbs and can have a Y-, X-, H-, or T-, cross-sectional shape with a symmetrical Y-shape as shown for the fibre 300 in Fig 3 being preferred.

Multilimbed viscose fibres are not as susceptible to compression as are non-limbed viscose fibres, or cellulosic fibres such as cotton fibres and fluffed pulp. When compressing a fibrous mass predominantly comprising multilimbed viscose fibres, the fibres will tend to return to a less compressed state, resulting in a less compact structure than when using fibres that are more compressible. The spring-back effect is believed to mainly depend on the fibres being stiffer and more resilient than non-limbed fibres. However, the amount and type of spin-finish coating on the fibres have also been found to affect the springiness and compressibility of the fibres.

The reduced compressibility of the multilimbed viscose fibres is directly associated with a greater absorbency of multilimbed viscose fibres as compared to corresponding non-limbed viscose fibres. The reduced compressibility is also beneficial to the softness of the outer surface of the tampon and provides an open porous structure that will quickly acquire liquid that reaches the tampon. Moreover, the multilimbed viscose fibres have superior expansion properties and ascertain that the tampon conforms to the vaginal space and intercepts fluid flow, thus diminishing the risk for leakage.

In addition to absorbent and non-absorbent fibres, the absorption body 102 may also comprise polymeric gelling materials, also known as superabsorbents, as well as bacteria inhibiting agents or other chemically active additives.

The cover 103 may be any suitable non-abrasive liquid permeable material. Preferably, the cover 103 is a nonwoven material which may be a spunbonded, carded or spunlaced web made of polypropylene, polyethylene, viscose, bicomponent fibres or any other suitable fibrous material. However, perforated plastic films, cast or knitted nettings or similar porous materials may also be used. In the shown embodiment, the cover 103 completely encloses the absorption body 102. However, the cover 103 may be omitted or may be applied over only a part of the absorption body 102. For technical reasons, it is common to only apply a cover 103 on the straight cylindrical portion of the tampon, leaving the ends 105, 106 free from cover material. It is also conceivable to apply the cover only at one or both ends. The cover may be treated with a lubricating agent or similar in order to facilitate insertion.

The withdrawal string 104 is made from a material having high tensile strength and is firmly attached to the tampon body by being looped internally between layers in the absorption body 102 or by being welded, glued or sewn to the absorption body 102 and/or to the liquid permeable cover 103. The withdrawal string 104 should not be absorbent or have any appreciable wicking capacity. In the embodiment shown in Fig. 1, the withdrawal string 104 is internally locked between layers in the tampon body 102 and protrudes with two ends from the withdrawal end 106 of the tampon. An alternative and commonly used way of locking a looped string to a tampon body is by tying the protruding ends together in a knot.

In Fig. 4 is shown a tampon blank 401 that can be used for forming a tampon in accordance with the invention. The tampon blank 401 comprises a first fibrous web or band 408 and a second fibrous web or band 409. In the finished, compressed tampon, the first band 408 forms the inner core and the second band 409 forms the outer absorbent structure. Accordingly, the fibrous compositions of the respective bands are as disclosed above in connection with Figs. 1 and 2. The bands can be made from carded staple length fibres or can be formed by other techniques such as air-laying, meltblowing, wet-laying, etc. As shown in Fig. 4, the bands 408, 409 can be of equal width. It is also conceivable that the bands 408, 409 have different widths. Hence, it may be beneficial to make the first band 408 somewhat narrower than the second band 409 if it is desirable that the tampon be given a rounded or tapered shape at one or both ends. Alternatively, the first band 408 can be made wider than the second band 409 if this is desired in order to aid in the fixation of the withdrawal string in the tampon.

The first band 408 is placed on the second band and overlaps a portion of an end of the second band 409. Moreover, the bands 408, 409 are attached to each other in the overlapping area 410. The attachment is shown as a number of spot bonds 411. The attachment can be made in any suitable manner such as by hot calandering, thermobonding, hydroentangling, needling, by spraying of an adhesive, etc. The attachment can be made by discrete bonds such as shown in Fig. 4 or can be a continuous bond.

A cover sheet 403 is arranged in an overlapping fashion at the opposite end of the second fibrous band 409. The cover sheet 403 is attached to the second fibrous band 409 in the overlapping area 412 by means of a fusion bond 413. Like the attachment between the first and second bands 408, 409, the attachment of the cover sheet 403 to the second fibrous band 409 can be made in any suitable manner and with any suitable bond pattern. However, conventional cover materials often comprise a relatively large proportion of thermoplastic material that can be used to weld the cover to the second fibrous band 409.

The cover sheet 403 is wider than the first and second fibrous bands 408, 409 forming extending portions 414 on either side of the bands 408, 409.

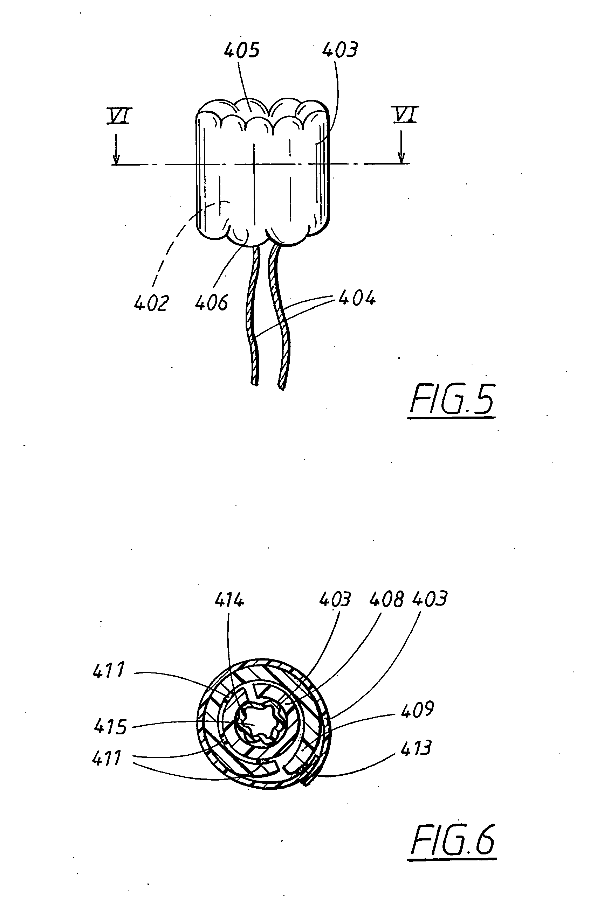

Figs. 5 and 6 show the tampon blank 401 with the attached cover 403 rolled up into an uncompressed tampon. The extending portions 414 of the cover sheet 403 have been tucked into the interior of the tampon at both ends 405, 406 thereof. In Fig. 5, the uncompressed tampon is shown to comprise a withdrawal string 404. However, the attachment of the withdrawal string is not shown in the Figs. In embodiments having the withdrawal string locked by internally looping it between layers in the tampon body, it can be beneficial to have a greater concentration of multi-limbed fibres at the surfaces of the first band than in its interior. As an effect of the spin-finish coating used on the multi-limbed viscose fibres, the multi-limbed fibres show a higher friction and, thus, serves better to prevent the withdrawal string from sliding between the layers.

As is best seen in Fig. 6, the rolled-up tampon has an inner cavity 415 into which the extending portions 414 of the cover sheet 403 are tucked. The first fibrous band 408 forms an inner tubular roll and the second fibrous band 409 is wound on the outer surface of the inner roll. The cover sheet 403 is wound on the outside of the second fibrous band 409 so as to enclose the rolled-up tampon blank.

As previously mentioned, there are numerous alternative ways to produce a tampon according to the invention. It is, for instance, possible to form and pre-compress the inner core in a separate production step. Depending on the production method, the attachment between the layers may be omitted or may be made in different locations from those described herein.

The invention is not limited to the embodiments disclosed herein. Accordingly, further modifications are conceivable within the scope of the appended claims.

## Claims

1. A tampon (101) having an insertion end (105) and a withdrawal end (106), and a withdrawal string (104) extending from the withdrawal end (106), said tampon (101) comprising a fibrous absorbent tampon body (102) comprising multi-limbed viscose fibres, said tampon body (102) comprising an inner core (108) and an outer absorbent structure (109) surrounding said inner core (108), said inner core (108) having a higher density than said outer absorbent structure (109) **characterised in that** said inner core (108) is made of a fibrous blend comprising 0 - 25% by weight of multilimbed viscose fibres and said outer absorbent structure (109) is made of a fibrous blend comprising 30 - 100% by weight of multilimbed viscose fibres.

2. A tampon according to claim 1, **characterised in that** said inner core (108) comprises a blend of 0 - 25% by weight of multilimbed viscose fibres and 75 - 100 % by weight of non-limbed viscose fibres.

3. A tampon according to claim 1 or 2, **characterised in that** the multilimbed viscose fibres have an absorbent capacity of at least 5 g/g and preferably of at least 5.5 g/g.

4. A tampon according to claim 1, 2, or 3, **characterised in that** the inner core (108) has a density of 0.40 - 0.60 g/cm³, preferably 0.50 - 0.55 g/cm³ and the outer absorbent structure (109) has a density of 0.30 - 0.45 g/cm³, preferably 0.35 - 0.40 g/cm³.

5. A tampon according to any one of the preceding claims, **characterised in that** the inner core (108) has an absorption capacity of at least 3,5 g/g and preferably at least 4 g/g and the outer absorbent structure (109) has an absorption capacity of at least 5 g/g and preferably of at least 5.5 g/g.

6. A tampon according to any one of the preceding claims, **characterised in that** the inner core (108) comprises at least 80% of non-limbed cellulosic fibres and the outer absorbent structure (109) comprises at least 80% multi-limbed viscose fibres.

7. A tampon according to any one of the preceding claims, **characterised in that** said outer absorbent structure (109) exhibits at least three longitudinally extending grooves (107) and at least three longitudinally extending ridges (116) separating the grooves (107).

8. A tampon according to any one of the preceding claims, **characterised in that** a liquid permeable cover sheet (103) is arranged on the surface of said absorbent tampon body (102).

9. A tampon according to claim 8, **characterised in that** said absorbent body is completely covered by said liquid permeable cover sheet (103).

10. A tampon according to claim 8 or 9, **characterised in that** a liquid barrier is arranged at the withdrawal end (106) of the tampon.

11. A tampon according to any one of the preceding claims, **characterised in that** the inner absorbent core (108) has an outer portion in contact with the outer absorbent structure (109) and that a higher concentration of multi-limbed viscose fibres is present in the outer portion of the inner absorbent core (108) than in an interior portion of the inner absorbent core (108).

## Patentansprüche

1. Tampon (101) mit einem Einführungsende (105) und einem Entnahmeende (106) und einem Entnahmefaden (104), der sich vom Entnahmeende (106) erstreckt, wobei der Tampon (101) einen absorbierenden Tamponfaserkörper (102), umfassend mehrgliedrige Viskosefasern, umfasst, wobei der Tamponkörper (102) einen inneren Kern (108) und eine äußere absorbierende, den inneren Kern (108) umgebende Struktur (109) umfasst, wobei der innere Kern (108) eine höhere Dichte als die äußere absorbierende Struktur (109) aufweist, **dadurch gekennzeichnet, dass** der innere Kern (108) aus einem Fasergemisch, umfassend 0 - 25 Gewichtsprozent mehrgliedrige Viskosefasern hergestellt ist, und die äußere absorbierende Struktur (109) aus einem Fasergemisch, umfassend 30 - 100% Gewichtsprozent mehrgliedrige Viskosefasern hergestellt ist.

2. Tampon nach Anspruch 1, **dadurch gekennzeichnet, dass** der innere Kern (108) ein Gemisch von 0 - 25 Gewichtsprozent mehrgliedrige Viskosefasern und 75 - 100 Gewichtsprozent nichtgliedrige Viskosefasern umfasst.

3. Tampon nach Anspruch 1 oder 2, **dadurch gekennzeichnet, dass** die mehrgliedrigen Viskosefasern eine Absorptionskapazität von mindestens 5 g/g und vorzugsweise von mindestens 5,5 g/g aufweisen.

4. Tampon nach Anspruch 1, 2 oder 3, **dadurch gekennzeichnet, dass** der innere Kern (108) eine Dichte von 0,40 - 0,60 g/cm³, vorzugsweise 0,50 - 0,55 g/cm³, und die äußere absorbierende Struktur (109) eine Dichte von 0,30 - 0,45 g/cm³, vorzugsweise 0,35 - 0,40 g/cm³, aufweist.

5. Tampon nach einem der vorgehenden Ansprüche, **dadurch gekennzeichnet, dass** der innere Kern (108) eine Absorptionskapazität von mindestens 3,5 g/g und vorzugsweise mindestens 4 g/g und die äußere absorbierende Struktur (109) eine Absorptionskapazität von mindestens 5 g/g und vorzugsweise mindestens 5,5 g/g aufweist.

6. Tampon nach einem der vorgehenden Ansprüche, **dadurch gekennzeichnet, dass** der innere Kern (108) mindestens 80% nichtgliedrige Zellulosefasern und die äußere absorbierende Struktur (109) mindestens 80% mehrgliedrige Viskosefasern umfasst.

7. Tampon nach einem der vorgehenden Ansprüche, **dadurch gekennzeichnet, dass** die äußere absorbierende Struktur (109) mindestens drei sich längserstreckende Rillen (107) und mindestens drei sich längserstreckende Erhöhungen (116), die die Rillen (107) trennen, aufweist.

8. Tampon nach einem der vorgehenden Ansprüche, **dadurch gekennzeichnet, dass** eine flüssigkeitsdurchlässige Abdeckschicht (103) auf der Oberfläche des absorbierenden Tamponkörpers (102) angeordnet ist.

9. Tampon nach Anspruch 8, **dadurch gekennzeichnet, dass** der absorbierende Körper von der flüssigkeitsdurchlässigen Abdeckung (103) vollkommen gedeckt ist.

10. Tampon nach Anspruch 8 oder 9, **dadurch gekennzeichnet, dass** eine Flüssigkeitsbarriere am Entnahmeende (106) des Tampons angeordnet ist.

11. Tampon nach einem der vorgehenden Ansprüche, **dadurch gekennzeichnet, dass** der innere absorbierende Kern (108) eine äußeren Teil aufweist, der mit der äußeren absorbierenden Struktur (109) in Kontakt ist, und dass eine höhere Konzentration von mehrgliedrigen Viskosefasern im äußeren Teil des inneren absorbierenden Kerns (108) als in einem inwendigen Teil des inneren absorbierenden Kerns (108) vorkommt.

## Revendications

1. Tampon (101) ayant une extrémité d'insertion (105) et une extrémité de retrait (106), et un cordon de retrait (104) s'étendant depuis l'extrémité de retrait (106), ledit tampon (101) comprenant un corps de tampon absorbant fibreux (102) comprenant des fibres de viscose à branches multiples, ledit corps de tampon (102) comprenant un noyau intérieur (108) et une structure absorbante extérieure (109) entourant ledit noyau intérieur (108), ledit noyau intérieur (108) ayant une densité supérieure à celle de ladite structure absorbante extérieure (109), **caractérisé en ce que** ledit noyau intérieur (108) est fait d'un mélange fibreux comprenant de 0 à 25% en poids de fibres de viscose à branches multiples, et ladite structure absorbante extérieure (109) est faite d'un mélange fibreux comprenant de 30 à 100% en poids de fibres de viscose à branches multiples.

2. Tampon selon la revendication 1, **caractérisé en ce que** ledit noyau intérieur (108) comprend un mélange de 0 à 25% en poids de fibres de viscose à branches multiples et de 75 à 100% en poids de fibres de viscose sans branches.

3. Tampon selon la revendication 1 ou 2, **caractérisé en ce que** les fibres de viscose à branches multiples ont une capacité d'absorption d'au moins 5 g/g et préférablement d'au moins 5,5 g/g.

4. Tampon selon la revendication 1, 2 ou 3, **caractérisé en ce que** le noyau intérieur (108) a une densité comprise entre 0,40 et 0,60 g/cm³, préférablement entre 0,50 et 0,55 g/cm³, et la structure absorbante extérieure (109) a une densité comprise entre 0,30 et 0,45 g/cm³, préférablement entre 0,35 et 0,40 g/cm³.

5. Tampon selon l'une quelconque des revendications précédentes, **caractérisé en ce que** le noyau intérieur (108) a une capacité d'absorption d'au moins 3,5 g/g et préférablement d'au moins 4 g/g, et la structure absorbante extérieure (109) a une capacité d'absorption d'au moins 5 g/g et préférablement d'au moins 5,5 g/g.

6. Tampon selon l'une quelconque des revendications précédentes, **caractérisé en ce que** le noyau intérieur (108) comprend au moins 80% de fibres cellulosiques sans branches, et la structure absorbante extérieure (109) comprend au moins 80% de fibres de viscose à branches multiples.

7. Tampon selon l'une quelconque des revendications précédentes, **caractérisé en ce que** ladite structure absorbante extérieure (109) présente au moins trois rainures (107) s'étendant longitudinalement et au moins trois nervures (116) s'étendant longitudinalement, qui séparent les rainures (107).

8. Tampon selon l'une quelconque des revendications précédentes, **caractérisé en ce qu'**une feuille de couverture perméable aux liquides (103) est disposée sur la surface dudit corps de tampon absorbant (102).

9. Tampon selon la revendication 8, **caractérisé en ce que** ledit corps absorbant est complètement recouvert par ladite feuille de couverture perméable aux liquides (103).

10. Tampon selon la revendication 8 ou 9, **caractérisé en ce qu'**une barrière aux liquides est disposée à l'extrémité de retrait (106) du tampon.

11. Tampon selon l'une quelconque des revendications précédentes, **caractérisé en ce que** le noyau absorbant intérieur (108) a une portion extérieure en contact avec la structure absorbante extérieure (109), et qu'une concentration plus élevée de fibres de viscose à branches multiples est présente dans la portion extérieure du noyau absorbant intérieur (108) que dans une portion intérieure du noyau absorbant intérieur (108).
